# EUROPEAN PATENT APPLICATION

(11) **EP 0 687 989 A2**
(43) Date of publication of application: **20.12.1995**
(21) Application number: 95850108.2
(22) Date of filing: 13.06.1995
(51) Int. Cl.: G06F 19/00

(54) **System for automated recording and retrieval of information of patients with patient data verification**

(30) Priority: 13.06.1994 US 259036
(71) Applicant: ALTHIN MEDICAL INC., Miami Lakes, Florida 33014 (US)
(72) Inventor: Ranslam, Robert E., Jr., Oregon City, OR 97045 (US); Kelly, Thomas D., Portland, OR 97225 (US); Merizan, Mark I., Sherwood, OR 97140 (US); Wolfe, Alan G., Portland, OR 97202 (US)
(74) Representative: Roth, Ernst Adolf Michael

(57) **Abstract**

A system for recording information related to dialysis treatments provides verification of patient-identifying data. Patient-identifying data, such as a patient ID number, is entered using a dialysis machine used to provide dialysis treatment. The system accesses and displays at the dialysis machine an associated patient-identifying datum, such as the patient name, along with a query requesting verification of correct patient-identifying data entry. On receiving an affirmative verification response, the dialysis machine provides treatment and the system records data related to the treatment in association with the patient-identifying data.

## Description

### FIELD OF THE INVENTION

This invention relates generally to systems for automated recording and retrieval of information on patients undergoing hemodialysis treatments, and more particularly relates to recall and verification of patient identification and treatment parameters before initiating a hemodialysis treatment of the patient.

### BACKGROUND AND SUMMARY OF THE INVENTION

Modern maintenance hemodialysis is typically performed in a clinical center to which patients periodically report several times a week to receive hemodialysis treatments. Such centers typically treat many patients, and each patient often receives, as prescribed, a treatment that differs in some respect from treatments received by other patients at the center. I.e., a number of parameters must normally be controlled during a hemodialysis treatment, and each patient usually has a distinctive parametrical profile associated with her treatment. To ensure proper quality control, the center should provide a way to make sure that each patient receives the particular treatment prescribed for the patient.

One way in which the foregoing is frequently done is to maintain a written treatment record for each patient. Before each treatment, the clinician manually consults the patient's file and sets up the hemodialysis machine for treating the patient according to the parameters set forth in the patient's file. During and after the treatment, the clinician prepares a written treatment report for the patient that is placed in the patient's file. The treatment report includes information on treatment parameters and other data that can be useful in future treatments of the patient. Whereas such a scheme can be effective, it is increasingly disfavored, particularly in busy clinics, because of the lengthy time required to consult the patient's file, prepare treatment reports, and manually set up the machine immediately before commencing treatment. Also, such manual systems are tedious and thus error-prone. Errors also arise due to the lack of any consistent way in such systems to ensure that the clinician has consulted the proper file for a particular patient.

To reduce the probability of errors and the time required to prepare for a particular patient's treatment, some clinics have automated patient record keeping by storing such records in a computer database. The clinician gains access to each patient's record by keying in, at the computer, a unique code number or word for the patient; this causes the computer to display certain information regarding treatment parameters for the patient. Patient code numbers usually consist of four or more digits (the patients' Social Security Numbers are frequently used.) Unfortunately, machine setup using such a scheme is still manual and is thus error-prone. Also, it is easy, particularly in a busy clinic, to mis-key a patient's code number, thereby causing the computer to store and/or display the wrong information for a particular patient.

The present invention provides systems and methods that overcome the foregoing and other drawbacks of previous systems. The instant system comprises a data storage and retrieval subsystem (termed herein the "Clinical Support Subsystem", which is abbreviated "CSS") and at least one hemodialysis machine.

The hemodialysis machine is preferably as disclosed in U.S. Patent No. 5,247,434, incorporated herein by reference. (The machine disclosed in said Patent is termed herein the "System 1000" or SYSTEM 1000 machine.) Thus, the hemodialysis machine is preferably of a type that permits the clinician user to "enter" (i.e., key-in) a patient identification number or code word as well as set up a treatment for a particular patient according to parameters set forth in the patient's treatment prescription.

According to the present invention, entry of a patient-identifying number or code word prior to dialysis treatment causes the system to search data in the Css for a corresponding patient name. The recalled patient name is then displayed on the hemodialysis machine along with a query requesting that the clinician verify the recalled name. If the recalled name is correct, the clinician "verifies" the recalled name by an appropriate response to the query. If the clinician has responded affirmatively to the query, further setup of the hemodialysis machine in preparation for treating the respective patient can be performed. If the query is not answered or is answered negatively, further setup cannot be performed.

Additional features and advantages of the invention will be made apparent from the following detailed description of a preferred embodiment which proceeds with reference to the accompanying drawings.

### BRIEF DESCRIPTION OF THE DRAWINGS

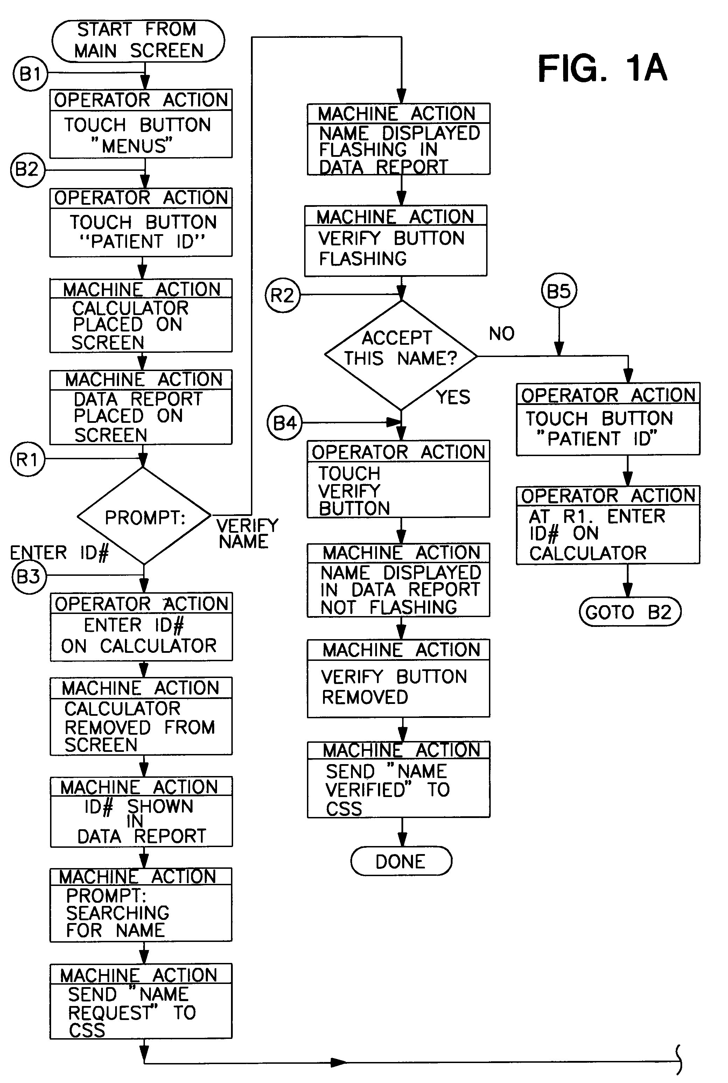

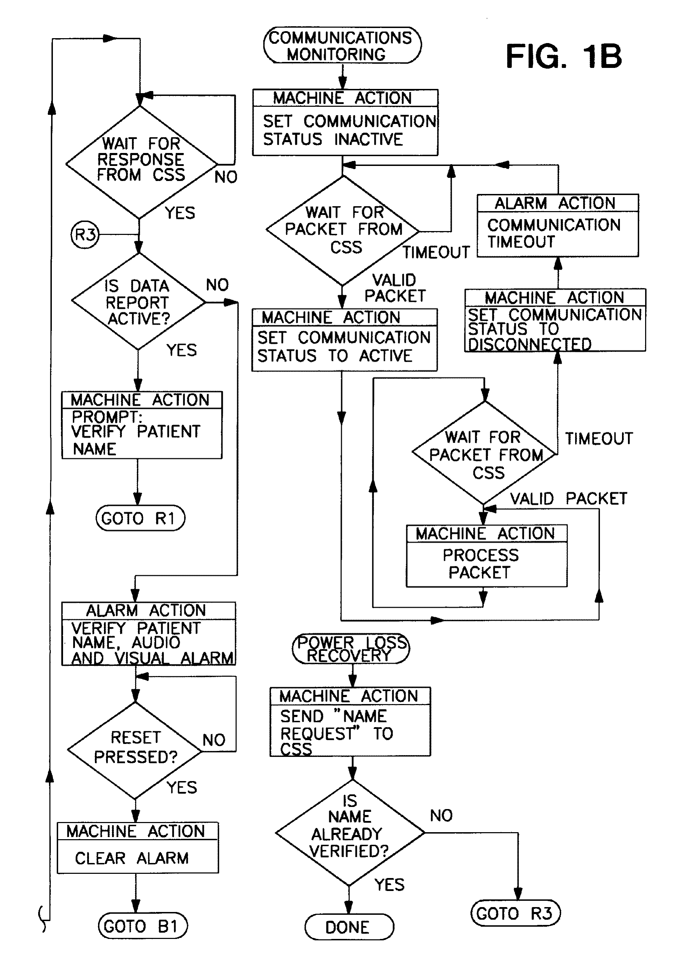

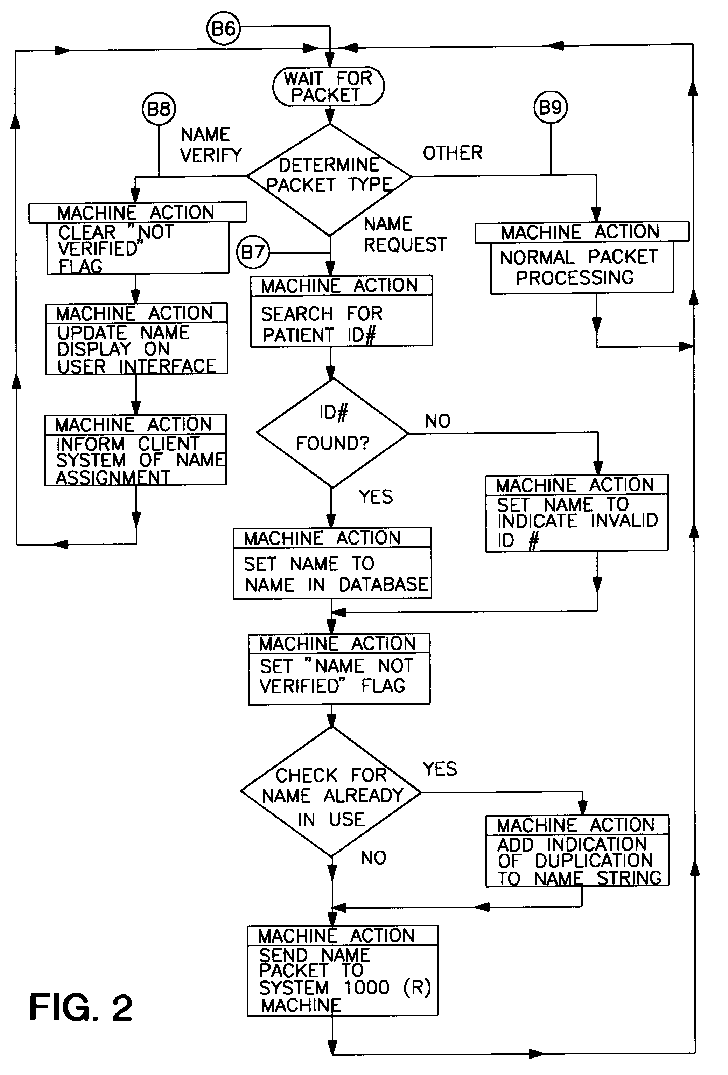

FIGS. 1 and 2 are flow charts of a process according to a preferred embodiment of the present invention for verifying patient identification.

### DETAILED DESCRIPTION OF A PREFERRED EMBODIMENT

A system according to the preferred embodiment of the invention comprises two subsystems. A first subsystem comprises at least one hemodialysis machine, preferably the System 1000® dialysis machine, a detailed disclosure of which is in U.S. Pat. No. 5,247,434, incorporated herein by reference. A second subsystem comprises a data storage and retrieval device, such as a microprocessor or computer, electrically connected to the hemodialysis machine. The data storage and retrieval subsystem is termed herein the "Clinical Support Subsystem", abbreviated "CSS".

### Entry and Verification of Patient Code Number

Entry and verification of a patient code number or word are as follows:

Each patient in the center is assigned a unique code number or word (also termed herein the "patient ID number") consisting of at least four characters such as four digits. (Four or more characters provides a sufficient number of unique code words for all the patients receiving treatment at a typical hemodialysis clinic.)

A patient code number is entered preferably by using a numerical keypad displayed on the SYSTEM 1000 touch screen. The entered code number is sent to the CSS. The CSS includes memory on which all code numbers and corresponding patient names are stored in the form of a database. The CSS is programmed with software that causes the CSS to scan the database to find the patient name (and other data) associated with the code number. If a corresponding patient name is thus "found", it is sent to the SYSTEM 1000 machine for display on the touch screen. A "verify" button is also displayed. If the user of the machine agrees that the displayed patient name corresponds to the entered code number, she touches the "verify" button, which sends a name-verify message to the CSS.

In more detail, referring to FIG. 1, the following events occur:
- B1: The user touches the MENUS button on the touch screen, which invokes a MENUS screen display on the touch screen. A "CSS data report" is displayed automatically (and remains displayed) on the touch screen. The screen includes a PATIENT ID button soliciting a response from the user.
- B2: The user touches the PATIENT ID button. The PATIENT ID button becomes highlighted on the touch screen. If the SYSTEM 1000 machine is in a "prime" or "dialyze" mode, or in an "inalarm" or "exalarm" state, a numerical keypad is invoked on the keypad to allow the user to key in a code number corresponding to a new patient. The CSS data report, including a patient's name, remains displayed on the touch screen.
- R1: A message "Enter New Patient ID" appears in a "prompt" region in the data report displayed on the touch screen, invoking the user to enter a new patient ID number or, alternatively, to verify the patient name displayed in the CSS data report. If the user wishes to invoke a display of a new patient name, the corresponding patient ID number is keyed-in using the keypad, followed by touching an ENTER button. If the user wishes to maintain display of the previous patient's name, the touch screen invokes the same by displaying a VERIFY PATIENT ID button.
- B3: After the user presses the ENTER button (after keying-in a new patient ID number), the keypad disappears from the touch screen; the just-entered patient ID number is displayed in the CSS data report; and a "Searching for Patient Name" message appears in the data report prompt area. Thus, the System 1000 issues a name request to the CSS, during which the PATIENT ID button remains highlighted.
- R2: Should the CSS "find" a patient name corresponding to the new patient ID number, the new name appears, flashing, in the CSS data report and a VERIFY PATIENT NAME button appears.
Thus, the user is prompted to confirm the correctness of the displayed patient name by touching the VERIFY PATIENT NAME button.
- B4: After being touched by the user, the VERIFY PATIENT NAME button disappears, and the PATIENT ID button remains highlighted. Also, the CSS data report remains displayed on the touch screen. At this time, the user can transition to the main screen by touching the MAIN SCREEN button. By touching the PATIENT ID button, the highlighting of said button would disappear, and the CSS data report is replaced by the main data report.
- B5: If the name displayed in the CSS data report, as "found" by the CSS to correspond to the keyed-in patient ID number, is incorrect, the user touches the PATIENT ID button, causing highlighting of the PATIENT ID button to disappear, the CSS data report to be removed, and appearance of the main data report on the MENUS screen. Thus, the user is prompted to touch the PATIENT ID button and repeat the process as described above.

### Communication Status:

Upon powerup, CSS communication status is "Inactive." Upon receipt of the first CSS communication, the status becomes "Active."

Should the SYSTEM 1000 machine not receive a CSS communication for a given amount of time, the CSS communication status goes from "Active" to "Disconnected." Also, an "error" condition will be arise to permit user identification of the loss of communication.

Upon a new communication from the CSS, the status will transition from "Disconnected" to "Active."

### Power loss or recovery:

Should the SYSTEM 1000 machine return from a power-loss condition in the "prime," "dialyze," "inalarm," or "exalarm" modes with an entered valid patient ID number, a patient name request will automatically be issued to CSS corresponding to the entered number. This request will be transmitted when the CSS communication status transitions from "Inactive" to "Active."

If the user had verified the patient name prior to power loss, upon receipt of the patient name the machine would send a "patient name verified" message to the CSS. Power-loss recovery of the patient name and number is transparent to the user.

If the user had entered a valid patient ID number but had not yet verified the patient name prior to power loss, the VERIFY PATIENT NAME sequence would be initiated upon receipt of the patient name from CSS.

### Miscellaneous

The patient ID number is transmitted in all communications.

The numerical keypad is invoked on the touch screen whenever the user transitions from the MENUS data report screen to the CSS data report screen, as described in B2, above. The keypad appears immediately, but will disappear after a time if no keypad buttons are touched. Should the keypad disappear due to the user not touching any of the keypad buttons, two touches of the PATIENT ID button will re-invoke display of the keypad. (The first touch removes the CSS data report and the second touch re-invokes the CSS data report along with the keypad.

### CSS Name Entry and Verification Description

Briefly, the CSS patient ID entry and verification can be described as follows:

The CSS system initiates and receives communications from one or more SYSTEM 1000 machines. When a patient ID number is entered using a SYSTEM 1000 touch screen, the number is transmitted to the CSS system. The CSS "searches" the CSS patient database for the patient name corresponding to the patient ID number, and, if a name is "found", transmits the name to the SYSTEM 1000 for display on the touch screen. A "verify" message is sent to the CSS system whenever the SYSTEM 1000 user accepts the patient name (by touching the VERIFY PATIENT NAME button).

Referring now to FIG. 2:
- B6: Until a signal comes in from a SYSTEM 1000 machine, wait. When the signal arrives, determine the correct course of action: if the signal is a "Name Request", update internal data, look up the name and send it back to the SYSTEM 1000 from which the signal came (B7); if the signal is a "Name Verify", update internal data and verify the name to the SYSTEM 1000 machine from which the signal came (B8); otherwise, handle the signal in another appropriate way (B9).
- B7: A "Name Request" was just received from a SYSTEM 1000 machine: search in the database to find a patient name associated with the entered patient ID number. Add this name to the active station list, prepare this name to be sent back to the requesting SYSTEM 1000 machine, and update the internal tables.
If the corresponding patient name found has already be requested from the CSS from another SYSTEM 1000 machine connected to the CSS, or if the name is not in the database, indicate this in the information being prepared to send to the originating SYSTEM 1000. Send the prepared information back to the originating SYSTEM 1000 station, then wait for more information (B6).
- B8: A "Name Verify" message was just received from a SYSTEM 1000 machine: update the internal database and the station list. Inform the Client system of the new information, then wait for more information (B6).
- B9: The information received from a SYSTEM 1000 machine is not a "patient name request" or a "patient name verify" signal. This could include treatment or machine information, etc. Handle as appropriate, then wait for more information (B6).

Additional details of the invention are described in the attached appendices: Appendix A, Clinical Support System User's Guide; Appendix B, Clinical Support System Software Requirements Specification; Appendix C, CSS CRC Review; Appendix D, CSS Software Specification - Communication Packet Specification; Appendix E, CSS Software Specification - CSS Database Design; Appendix F, CSS Software Specification - System 1000 (R) Software Specification; and Appendix G, CSS Software Specification - HyperChart Communication.

Having described and illustrated the principles of our invention with reference to a preferred embodiment, it will be recognized that the preferred embodiment can be modified in arrangement and detail without departing from such principles. In view of the many possible embodiments to which the principles of our invention may be put, it should be recognized that the detailed embodiments are illustrative only and should not be taken as limiting the scope of our invention. Rather, we claim as our invention all such embodiments as may come within the scope and spirit of the following claims and equivalents thereto.

## Claims

1. A system for verifying entry of a patient identifying datum and for recording dialysis treatment information, comprising:
a dialysis machine for providing dialysis treatment to a patient;
a data-entry device in the dialysis machine for receiving entry of a patient-identifying datum;
a data-retrieval device in communication with the data-entry device for storing patient records, the data-retrieval device being operative to provide a patient-verifying datum associated with the patient-identifying datum;
an output device in the dialysis machine and in communication with the data-retrieval device for presenting the patient-verifying datum and a verification query in a visually perceptible form;
the data-entry device being operative to receive entry of one of an affirmative or negative datum responding to the verification query;
the dialysis machine providing the dialysis treatment to the patient upon entry of an affirmative datum, and generating treatment data related to the dialysis treatment; and
the data-retrieval device recording the treatment data in association with the patient-identifying datum.
